# EUROPEAN PATENT SPECIFICATION

(11) **EP 1 507 559 B1**
(45) Date of publication and mention of the grant of the patent: **26.07.2017**
(21) Application number: 03747660.3
(22) Date of filing: 02.05.2003
(51) Int. Cl.: A61K 9/16, A61K 47/69, A61P 41/00, A61P 35/00, A61K 31/337, A61K 31/436, A61K 31/7088

(54) **DELIVERY OF MICROPARTICLE-CONJUGATED DRUGS FOR INHIBITION OF STENOSIS**
ABGABE VON MIKROTEILCHEN-KONJUGIERTEN ARZNEIMITTELN ZUR HEMMUNG VON STENOSE
ADMINISTRATION DE MEDICAMENTS CONJUGUES A DES MICRO-PARTICULES AUX FINS D'INHIBITION D'UNE STENOSE

(30) Priority: 03.05.2002 US 138589; 02.07.2002 US 190419
(43) Date of publication of application: 23.02.2005
(73) Proprietor: Sarepta Therapeutics, Inc., Cambridge, MA 02142 (US)
(72) Inventor: IVERSEN, Patrick L., Corvallis, OR 97330 (US); KIPSHIDZE, Nicholas, New York, NY 10021 (US)
(74) Representative: Schiweck, Weinzierl & Koch Patentanwälte Partnerschaft mbB
(86) International application number: PCT/US2003/013892
(87) International publication number: WO 2003/092741

(56) References cited:
- WO-A1-00/02588
- WO-A1-99/00113
- WO-A1-02/087545
- WO-A2-98/00172
- US-A- 5 536 729
- US-A- 5 756 673
- US-A- 6 117 858
- US-B1- 6 273 913
- DAS G S ET AL: "CONTROLLED DELIVERY OF TAXOL FROM POLY(ETHYLENE GLYCOL)-COATED POLY(LACTIC ACID) MICROSPHERES" JOURNAL OF BIOMEDICAL MATERIALS RESEARCH, WILEY, NEW YORK, NY, US LNKD- DOI:10.1002/1097-4636(200104)55:1<96::AID- JBM130>3.0.CO;2-3, vol. 55, no. 1, 1 April 2001 (2001-04-01), pages 96-103, XP009004593 ISSN: 0021-9304

## Description

### Field of the Invention

The present invention relates to uses of a composition comprising an antirestenotic compound for the preparation of pharmaceutical compositions for treating or preventing hyperproliferative disease, e.g. stenosis, in blood vessels, and in particular to preventing stenosis following vascular injury, by delivery of a microparticle-conjugated antirestenotic drug being rapamycin, to a site of vascular injury.

### References

Albiero R et al, "Short- and intermediate-term results of 32P radioactive b-emitting stent implantation in patients with coronary artery disease." Circulation 101:18-26 (2000).
Barath P et al, Cathet Cardiovasc Diagn, Jul 1997, 41(3):333-41.
Bartorelli AL et al, Cathet Cardiovasc Diag, Nov 1997, 42(3):313-20.
CasterellaPJefa/., Cardiol Rev, Jul-Aug 1999, 7(4):219-31.
Cleland JL, Biotech Progress, Jan-Feb 1998, 14(1): 102-7.
Dev NB et al, Cathet Cardiovasc Diagn, Nov 1998, 45(3):337-45.
Dick A et al, Cardiovasc InterventRadiol, Sep-Oct 1999, 22(5):389-93.
Drachman DE et al, "Neointimal thickening after stent delivery of paclitaxel: change in composition and arrest of growth over six months." J Am Coll Cardiol 2000; 36:2325-32.
Edelman ER, Rogers C, "Pathobiologic responses to stenting." Am J Cardiol 1998, 81:4E-6E.
Fischman DL, Leon MB, Bairn DS et al, "A randomized comparison of coronary stent placement and balloon angioplasty in the treatment of coronary artery disease." Stent Restenosis Study Investigators. N Engl J Med. 1994, 331: 496-501.
Gottman D et al, Rofo Fortschr Geb Rontgenstr Neuen Bildgeb Verfahr, Jan 1999, 170(1):84-8.
Herdeg C, Oberhoff M, Baumbach A et al., "Local Paclitaxel delivery for the prevention of restenosis: biological effects and efficacy in vivo. "J Am Coll Cardiol. 2000; 35(7): 1969-76.
Herdeg C et al, Cathet Cardiovasc Diagn, Jul 1997, 41(3):308-14.
Herrmann SM et al, "Polymorphisms of the human matrix gla-protein gene (MGP); vascular calcification and myocardial infarction." Arterioscler Thromb Vase Biol 2000; 20: 2836-93.
Hiatt BL et al, Rev. Cardiovasc Medicine 2(4): 190-196 (2001).
Hodgkin DD et al., J Cardiovasc Pharmacol, Jan 1997, 29(1):39-44.
Iversen P. and Weller D., PCT Pubn. No. WO 00/44897, "Method of Treating Restenosis by Antisense Targeting of c-myc." (Aug 3 2000).
Kinsella JL and Sollot SJ, "Method of Treating Atherosclerosis or Restenosis Using Microtubule Stabilizing Agent", U.S. Patent No. 6,403,635 (June 11 2002).
Kipshidze N., Keane D. et al., Catheter Cardiac. Interv. 54(2):247-56 (Oct 2001).
Koh WJ et al., Int J Radiat Oncol Biol Phys., Nov 1 1996, 36(4):829-34.
Kornowski R, Hong MK, Tio FO et al., "In-stent restenosis: contributions of inflammatory responses and arterial injury to neointimal hyperplasia." J Am Coll Cardiol 1998; 31:224-230.
Kreuter J, J Anatomy, Dec 1996, 189(Pt 3):503-5.
Kwon GS, Crit Rev In Therap Drug Carrier Systems 1998, 15(5):481-512.
Leon MB, Teirstein PS, Moses JW et al., "Localized intracoronary gamma-radiation therapy to inhibit the recurrence of restenosis after stenting." N Engl J Med 2001; 25; 344(4):250-6.
Malhotra S, Teirstein PS, "The SCRIPPS trial -catheter-based radiotherapy to inhibit coronary restenosis." J Invasive Cardiol. 2000; 12(6): 330-32.
Morris RE and Gregory CR, "Method of Treating Restenosis with Rapamycin",U.S. Patent No. 5,516,781 (May 14 1996).
Oberhoff M et al., Cathet Cardiovasc Diagn, Jul 1997, 41(3):268-74.
Pavlides GS et al., Cathet Cardiovasc Diagn, Jul 1997, 41(3):287-92.
Porter TR et al., J Ultrasound Med, Aug 1996, 15(8):577.
Quintanar-Guerrero D et al., Drug Dev Ind Pharm Dec 1998, 24(12): 1113-28.
Raman VK et al., Semin Interv Cardiol, Sep-Dec 1998, 3(3-4):133-7.
Ravi Kumar MN, J Pharm & Pharm Sci May-Aug 2000, 3(2):234-58.
Robinson KA et al., Cathet Cardiovasc Diagn, Jul 1997, 41(3):348-53.
Roy S et al., J Vasc Interv Radiol Jun 1999, 10(6):817-24.
Rubartelli P et al., JAm Coll Cardiol, Jul 1998, 32(1):90-6.
Savage MP et al., J Am Coll Cardiol, Feb 1998, 31(2):307-11.
Serruys PW, de Jaegere P, Kiemeneij F et al., "A comparison of balloon-expandable stent implantation with balloon angioplasty in patients with coronary artery disease." N Eng J Med. 1994, 331: 489-495.
Soppimath, KS et al., J Controlled Release Jan 29 2001, 70(1-2):1-20.
Suzuki T et al., "Stent-based delivery of sirolimus reduces neo-intimal formation in a porcine coronary model." Circulation Sep 4 2001;104(10):1188-93.
Teomim D et al., J Controlled Release Jun 28 1999, 60(1): 129-42.

### Background of the Invention

Transluminal coronary angioplasty was introduced in the late 1970's as a nonsurgical treatment for obstructive coronary artery disease. Typically, the procedure involves placing a balloon-tip catheter at the site of occlusion, and disrupting and expanding the occluded vessel by inflating the catheter balloon. Since its introduction, major advances in equipment and techniques have led to widespread use of the method for treating coronary artery disease and angina. Recent studies have reported an equivalent seven-year survival rate for percutaneous transluminal coronary angioplasty (PTCA) and bypass surgery in patients with multivessel coronary artery disease. The process, however, damages the blood vessel wall, including loss of the endothelial lining of the vessel. Frequently the response to this injury includes myointimal hyperplasia, proliferation of fibroblasts, connective tissue matrix remodelling and formation of thrombus. These events lead to restenosis of the blood vessel, a segmentally limited, wound healing response to trauma of the vascular wall. This healing response leads to narrowing of the lumen of the vessel wall and hence to a high incidence (30 to 50%) of restenosis (Fischman *et al*., Serruys *et al.*).

Clinical trials in restenosis prevention using various revascularization devices, antiplatelet drugs, antithrombotic drugs, and anti-inflammatory agents have produced limited improvement in the incidence of restenosis. Attempts to improve the risk or severity of restenosis have employed intravascular stents (*e.g*. Savage, Rubarteli, Gottman), radiation therapy (Koh), and/or administration of anti-proliferative drugs at the vessel injury site. The latter approach typically employs the balloon catheter for introducing the therapeutic agent at the vessel occlusion site (Dick, Roy, Dev, Alfke, Robinson, Barath, Herdeg, Pavlides, Oberhoff, Hodgkin), or releasing drug from an implanted stent (Teomin, Bartonelli, Raman).

The use of coronary stent implantation has reduced the rate of angiographic restenosis to the low teens in large arteries. Coronary stents provide luminal scaffolding that virtually eliminates elastic recoil and remodeling. Stents, however, do not decrease neointimal hyperplasia and in fact lead to an increase in the proliferative comportment of restenosis (Edelman *et al.*)*.*

Drug coated or drug impregnated stents deployed within the lumen of the blood vessel have been widely explored as drug delivery devices. The drug is gradually eluted from the stent and diffuses into the vessel wall from the intima. Examples of drugs used to coat stents include rapamycin (Sirolimus®, Wyeth Ayerst), a macrolide antibiotic with immunosuppressive properties, paclitaxel (Taxol^{®,} Bristol-Myers Squibb), and actinomycin D, both chemotherapeutic agents. All of these have been shown to inhibit smooth muscle cell proliferation in such settings (Kinsella et al., 2002; *Herdeg et al*., 2000; Suzuki *et al*., 2001; Drachman *et al*., 2000; Hiatt *et al*., 2001).

However, with increased use of stent implantation, the frequency of in-stent restenosis also increases. There is evidence that the degree of inflammation and subsequent neointima formation is proportional to the degree of penetration of the vessel wall by the stent struts (Herdeg *et al.*)*.* Regardless of treatment strategy (*e.g.* PTCA, rotational atherectomy, laser angioplasty, cutting balloon angioplasty, or repeat stenting), the restenosis in case of in-stent restenosis is unacceptably high (20 to 80%).

Other limitations of drug-eluting stents include limitation of drug loading capacity and poor control of drug elution, resulting in unreliable pharmacokinetics. The devices are typically coated with biocompatible polymers, and durability of the polymer coatings has been problematic. The thickness of some currently used coatings makes these devices unsuitable for very small vessels. Finally, most of the current coatings are prone to causing chronic inflammatory responses. Other long term effects of the devices can include late thrombosis, weakening of the vessel wall, or delayed restenosis. Thus, long term follow-up is necessary to monitor the polymer's potential toxicity. Treatment with coated stents can also be costly, especially in cases where a multi-stenting procedure is planned.

Intracoronary brachytherapy (Leon *et al*., Malhotra *et al.*) is another current approach to prevention of renarrowing of an artery after angioplasty or stent placement. A small amount of radiation is delivered to the treated area, either via catheter, which delivers radiation to the treated area and is then removed, or via a radiation-emitting stent, which remains in place. Although shown to be effective in reducing the need for additional treatment of in-stent restenosis, the procedure may be associated with other complications. Weeks to months after brachytherapy, restenosis may occur at the edges of the treatment areas. Low-level radiation that penetrates beyond the targeted treatment area increases the growth of the soft tissue, resulting in narrowing, which is known as the "candy wrapper" or "edge" effect (Albiero *et al.*)*.* Such edge effects can also occur with drug-eluting stents, since the drug is not available beyond the edges of the stent.

Vascular occlusive phenomena also occur in other therapeutic settings. Autologous vein grafting, for example, is widely employed in coronary bypass procedures. About 400,000 to 500,000 first-time coronary graft procedures are performed every year in the United States alone. Although patient survival rates exceed 90% over the first five years after treatment, about 20% to 40% of the grafts fail during this time due to occlusive phenomena. Thus, 80,000-100,000 graft replacement procedures are needed in the U.S. yearly to avoid premature mortality.

Vascular occlusive phenomena also lead to failures in other vascular grafts, such as arterial-venous anastomosis used for kidney dialysis, and in organ transplants. In the vascular access model of kidney dialysis, a surgically formed arterial-venous anastomosis or shunt provides access to the artery and vein used for dialysis. During dialysis, the rate of blood flow, turbulence and stress at the venous junction is much higher than in a normal vein. Repeated exposure to these pressures frequently leads to hyperplasia and stenosis within the vein, causing dialysis access failure.

Accordingly, the incidence of restenosis, and the inability to predict the response to treatment, remains a serious risk factor in vascular angioplasty and other vascular surgical procedures.

WO 00/02588 A1 is directed to targeted site specific drug delivery compositions, achieving site specific delivery of a therapeutic substance, in particular for oligonucleotides.

Das et al., Journal of Biomedical Materials Research, Wiley, New York, vol. 55, no. 1, 1 April 2001, p. 96-103 discloses controlled delivery of taxol from poly(ethyleneglycol)-coated poly(lactic acid) microspheres.

WO 99/00113 A1 pertains to compositions and methods useful for the *in vivo* delivery of substantially water insoluble pharmacologically active agents in which the pharmacologically active agent is delivered in the form of suspended particles coated with protein.

US 5,536,729 discloses oral rapamycin formulations, in particular micellar formulations of rapamycin or a pharmaceutically acceptable salt thereof.

WO 02/087545 A1 discloses compositions and methods for the treatment of hyperplasia.

### Summary of the Invention

The present invention includes, in one aspect, a use of a composition comprising an antirestenotic compound for the preparation of a pharmaceutical composition for inhibiting stenosis formation in a blood vessel. Such stenosis typically results from trauma to a vessel, such as an incision, an angioplasty procedure, or other excessive pressure. In accordance with the use, a composition comprising an antirestenotic compound non-covalently conjugated to a microparticle carrier is administered to a site of trauma in the vessel. The antirestenotic compound is rapamycin.

The microparticle carrier comprises a suspension of insoluble gas-containing microbubbles in a pharmaceutically acceptable liquid vehicle, and is effective to deliver the conjugated therapeutic to the site of vessel trauma. The composition may be administered prior to, during, and/or following a procedure selected from balloon angioplasty, stent implantation, and surgical incision or grafting of the vessel. In one embodiment, where the procedure comprises stent implantation, administration of the composition is effective to inhibit intrastent restenosis; that is, restenosis distal and proximal to the stent.

Preferably, the therapeutic compound is released at the site of trauma without application of external stimulation (such as ultrasound or heat) to the composition following administration.
According to the invention the antirestenotic compound is rapamycin. The composition may further comprise, also conjugated to the microparticle carrier, an anti-inflammatory compound, e.g. a steroid such as dexamethasone, and/or a compound effective to inhibit collagen accumulation or calcification of the vascular wall.

In one embodiment, the carrier is a suspension of insoluble gas-containing microbubbles, where the gas is preferably SF₆ or a perfluorocarbon gas such as perfluoromethane, perfluoroethane, perfluoropropane, perfluorobutane, or perfluoropentane. The liquid vehicle is preferably an aqueous vehicle containing at least one filmogenic compound selected from a protein, surfactant, lipid, polysaccharide, and combinations thereof. In one embodiment, the liquid vehicle is an aqueous solution of human serum albumin and dextrose.

In a related aspect, the invention provides a use of a composition comprising an antirestenotic compound for the preparation of a pharmaceutical composition for inhibiting intrastent stenosis formation at regions proximal and distal to an implanted stent in a blood vessel, by administering to the vessel, prior to, during, and/or following implantation of the stent, a composition comprising an antirestenotic compound conjugated to a microparticle carrier, where the carrier comprises a suspension of insoluble gas-containing microbubbles, such as perfluorocarbon gas-containing microbubbles, in a pharmaceutically acceptable liquid vehicle. The antirestenotic compound is rapamycin. Administration is preferably carried out without application of external stimulation to the composition, during or following administration.

### Brief Description of the Drawing

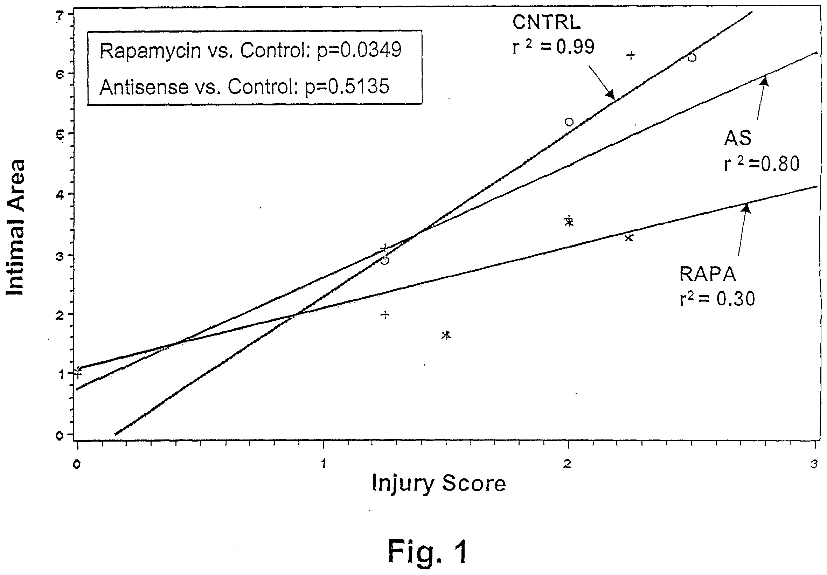

Figure 1 is a regression plot of IA (Intimal Area) vs. IS (Injury Score) determined from histomorphometric analysis of vessels in three groups of pigs which underwent balloon angioplasty and stent implantation, followed by treatment with microbubble-conjugated rapamycin, microbubble-conjugated *c-myc* antisense, or vehicle control.

### Detailed Description of the Invention

### I. Therapeutic Compositions

### A. Carrier Compositions

The present therapeutic compositions comprise a drug which is conjugated to a microparticle carrier, such as a gaseous microbubble in a fluid medium or a polymeric microparticle, with sufficient stability that the drug can be carried to and released at a site of vascular injury in a subject. Such conjugation typically refers to noncovalent binding or other association of the drug with the particle, and may be brought about by incubation with a microbubble suspension, as described further below, or intimate mixing of the drug with a polymeric microparticle carrier.

In one embodiment, the pharmaceutical composition comprises a liquid suspension, preferably an aqueous suspension, of microbubbles containing a blood-insoluble gas. The microbubbles are preferably about 0.1 to 10 µ in diameter. Generally, any blood-insoluble gas which is nontoxic and gaseous at body temperature can be used. The insoluble gas should have a diffusion coefficient and blood solubility lower than nitrogen or oxygen, which diffuse in the internal atmosphere of the blood vessel. Examples of useful gases are the noble gases, e.g. helium or argon, as well as fluorocarbon gases and sulfur hexafluoride. Generally, perfluorocarbon gases, such as perfluoromethane, perfluoroethane, perfluoropropane, perfluorobutane, and perfluoropentane, are preferred. It is believed that the cell membrane fluidizing feature of the perfluorobutane gas enhances cell entry for drugs on the surface of bubbles that come into contact with denuded vessel surfaces, as described further below.

The gaseous microbubbles are stabilized by a fluid filmogenic coating, to prevent coalescence and to provide an interface for binding of molecules to the microbubbles. The fluid is preferably an aqueous solution or suspension of one or more components selected from proteins, surfactants, lipids, including phospholipids, and polysaccharides. In preferred embodiments, the components are selected from proteins, surfactant compounds, and polysaccharides. Suitable proteins include, for example, albumin, gamma globulin, apotransferrin, hemoglobin, collagen, and urease. Human proteins, e.g. human serum albumin (HSA), are preferred.

Conventional surfactants include compounds such as alkyl polyether alcohols, alkylphenol polyether alcohols, and alcohol ethoxylates, having higher alkyl (*e.g.* 6-20 carbon atom) groups, fatty acid alkanolamides or alkylene oxide adducts thereof, and fatty acid glycerol monoesters. Surfactants particularly intended for use in microbubble contrast agent compositions are disclosed, for example, in Nycomed Imaging patents US 6,274,120 (fatty acids, polyhydroxyalkyl esters such as esters of pentaerythritol, ethylene glycol or glycerol, fatty alcohols and amines, and esters or amides thereof, lipophilic aldehydes and ketones; lipophilic derivatives of sugars, etc.), US 5,990,263 (methoxy-terminated PEG acylated with e.g. 6-hexadecanoyloxyhexadecanoyl), and US 5,919,434.

Other filmogenic synthetic polymers may also be used; see, for example, U.S. Patent Nos. 6,068,857 (Weitschies) and 6,143,276 (Unger), which describe microbubbles having a biodegradable polymer shell, where the polymer is selected from e.g. polylactic acid, an acrylate polymer, polyacrylamide, polycyanoacrylate, a polyester, polyether, polyamide, polysiloxane, polycarbonate, or polyphosphazene, and various combinations of copolymers thereof, such as a lactic acid-glycolic acid copolymer.

Such compositions have been used as contrast agents for diagnostic ultrasound, and have also been described for therapeutic applications, such as enhancement of drug penetration (Tachibana et al., U.S. Patent No. 5,315,998), as thrombolytics (Porter, U.S. Patent No. 5,648,098), and for drug delivery (see below). The latter reports require some external method of releasing the drug at the site of delivery, typically by raising the temperature to induce a phase change (Unger, U.S. Patent No. 6,143,276) or by exposing the microbubbles to ultrasound (Unger, U.S. Patent No. 6,143,276; Klaveness et al., U.S. Patent No. 6,261,537; Lindler *et al*., cited below, Unger *et al*., cited below; Porter et al., U.S. Patent No. 6,117,858).

In one embodiment, the carrier is a suspension of perfluorocarbon-containing dextrose/albumin microbubbles known as PESDA (perfluorocarbon-exposed sonicated dextrose/albumin). Human serum albumin (HSA) is easily metabolized within the body and has been widely used as a contrast agent. The composition may be prepared as described in co-owned U.S. Patents 5,849,727 and 6,117,858. Briefly, a dextrose/albumin solution is sonicated while being perfused with the perfluorocarbon gas. The microbubbles are preferably formed in an N₂-depleted, preferably N₂-free, environment, typically by introducing an N₂-depleted (in comparison to room air) or N₂-free gas into the interface between the sonicating horn and the solution. Microbubbles formed in this way are found to be significantly smaller and stabler than those formed in the presence of room air. (See *e.g.* Porter et al., U.S. Patent No. 6,245,747.)

The microbubbles are conjugated with rapamycin in the context of the present invention or another suitable immunosuppressive and/or antiproliferative drug in the context of the disclosure below. Generally, the microbubble suspension is incubated, with agitation if necessary, with a liquid formulation of the drug. Preferably, the liquid formulation of the drug(s) is first filtered through a micropore filter and/or sterilized. Drugs with limited aqueous solubility (which include rapamycin, tacrolimus, and paclitaxel) can be solubilized or intimately dispersed in pharmaceutically acceptable vehicles by methods known in the pharmaceutical arts. For example, rapamycin can be dissolved in, for example, alcohol, DMSO, or an oil such as castor oil or Cremophor™. A liquid formulation of rapamycin is also available from Wyett Ayerst Pharmaceuticals, and can be used for conjugation, preferably after sterilization with gamma radiation. Other solubilizing formulations are known in the art; see, for example, U.S. Patent No. 6,267,985 (Chen and Patel, 2001), which discloses formulations containing triglycerides and a combination of surfactants.

The incubation may be carried out at room temperature, or at moderately higher temperatures, as long as the stability of the drug or the microbubbles is not compromised. The therapeutic compound is believed to bind non-covalently at the gas/fluid interface of the microbubbles. The extent of binding of the compound may be evaluated, if desired, by partitioning of labeled compound between the microbubble suspension and a solvent, as described, for example, in PCT Pubn. No. WO 98/00172.

Microbubble-therapeutic compositions have also been previously described in, for example, U.S. Patent No. 6,143,276 (Unger), U.S. Patent No. 6,261,537 (Klaveness et al.), Lindler et al., Echocardiography 18(4):329, May 2001, Unger et al., Echocardiography 18(4):355, May 2001, and Price et al., J. Cardiovasc. Pharmacol. Ther. 7(3):171-80, July 2002. In these references, the compounds are released from the microbubbles *in vivo* by application of ultrasound at the desired point of release. Targeting of tumors by lipid-coated microbubbles has been described by Ho et al., Neurosurgery 40(6):1260-6, Jun 1997.

As described herein, neither ultrasound, nor other external stimulation, was required for delivery of therapeutically effective amounts of rapamycin from microbubbles to damaged endothelium in angioplasty-injured coronary vessels.

In addition to gas-filled microbubbles, other biocompatible microparticles, preferably polymeric particles, may also be used for delivery of a conjugated drug, *e.g*. rapamycin, to damaged endothelium, since very small particles tend to adhere to denuded vessel surfaces (*i.e.* vessels having damaged endothelium).

In this sense, "nanoparticles" refers to particles in the nanometer size range (*e.g.* 50 to 750 nm), while "microparticles" refers to particles in the micrometer size range (*e.g*. 1 to 50 µ), but may also include particles in the submicromolar range, down to about 0.1 µ. For use in the methods described herein, a size range of about 0.1 to 10 µ is preferred.

Polymeric particles have been described for use as drug carriers into which drugs or antigens may be incorporated in the form of solid solutions or solid dispersions, or onto which these materials may be absorbed or chemically bound. See *e.g*. Kreuter 1996; Ravi Kumar 2000; Kwon 1998. Methods for their preparation include emulsification evaporation, solvent displacement, "salting-out", and emulsification diffusion (Soppimath *et al*.; Quintanar-Guerrero *et al.*), as well as direct polymerization (Douglas *et al.*) and solvent evaporation processes (Cleland).

Preferably, the polymer is bioerodible *in vivo.* Biocompatible and bioerodible polymers that have been used in the art include poly(lactide-co-glycolide) copolymers, polyanhydrides, and poly(phosphoesters). Poly(orthoester) polymers designed for drug delivery, available from A.P. Pharma, Inc., are described in Heller et al., J. Controlled Release 78(1-3):133-141 (2002). In one embodiment, the polymer is a diol - diol monoglycolide - orthoester copolymer. The polymer can be produced in powdered form, *e.g.* by cryogrinding or spray drying, intimately mixed in powdered form with a therapeutic compound, and fabricated into various forms, including microspheres and nanospheres.

### B. Therapeutic Compounds

The therapeutic compositions include at least one antirestenotic agent, preferably an immunosuppressive, antiproliferative, and/or anti-inflammatory drug, conjugated to and delivered by the carrier composition described above. Examples of drugs with significant antiproliferative and/or immunomodulating effects include rapamycin, paclitaxel and other taxanes, tacrolimus, angiopeptin, flavoperidol, actinomycin D, methotrexate, angiopepsin, mitomycin, cyclosporin, leflunomide, mycophenolic acid, ritonavir, mizoribine, tranilast, and active analogs, derivatives or prodrugs of these compounds. One example of such a derivative is everolimus, the 42-O-(2-hydroxyethyl) derivative of rapamycin. In the present application, rapamycin is the antirestenotic compound used in the compositions. Also included are antisense oligonucleotides having antiproliferative effects, such as oligonucleotides effective to inhibit expression of genes encoding enzymes which mediate smooth muscle proliferation. Preferred oligonucleotide analogs include morpholino-based oligomers having uncharged, phosphorus-containing linkages, as described, for example, in U.S. Patent Nos. 5,185,444 and 5,142,047. Oligonucleotides antisense to *c-myc* may be used, and preferably include those described in copending and co-owned application having U.S. serial no. 09/493,427, and in PCT Pubn. No. WO 00/44897 and U.S. Appn. Pubn. No. 20010024783.

Other therapeutic agents that may be used beneficially to inhibit restenosis include anti-inflammatory steroids, such as dexamethasone or prednisoline; vassenoids; hormones such as estrogen; matrix metalloproteinase inhibitors, such as batimastat; protease inhibitors; lipid lowering compounds; ribozymes, such as PCNA ribozyme; vascular, bone marrow and stem cells; diltiazem; acridine; clopidogrel; antithrombins; anticoagulants, such as heparin or hirudin; and migration inhibitors, such as halofuginone and probucol. Also included are antioxidants; antiplatelets; IIBIIIA antagonists; antibiotics; calcium channel blockers; converting enzyme inhibitors; cytokine inhibitors; growth factors; growth factor inhibitors; growth factor sequestering agents; tissue factor inhibitors; smooth muscle inhibitors; organoselenium compounds; retinoic acid and other retinoid compounds; sulfated proteoglycans; superoxide dismutase mimics; and compounds that promote vascular healing and re-endothelialization, such as NO, NO precursors, and VEGF (vascular endothelial growth factor). Combinations of two or more therapeutic compounds may be used.

The compositions of the invention may also include agents, preferably in combination with an antiproliferative agent, that inhibit collagen accumulation and/or calcification of the vascular wall. For example, local delivery of vitamin K has been'reported to counteract the calcification effect associated with vessel injury (Herrmann *et al,* 2000).

Agents believed to function via different antirestenotic mechanisms may be expected to act synergistically. It may be useful, therefore, to combine two or more of these agents; e.g. to combine an antiproliferative and/or immunosuppressive agent with an anti-inflammatory and/or an anticalcification agent.

The therapeutic agent conjugated to the microparticles is preferably selected from the group consisting of rapamycin (sirolimus), tacrolimus (FK506), paclitaxel (Taxol®), epothilone D, fractionated or unfractionated heparin, and flavoperidol, as well as active analogs or derivatives, such as prodrugs, of these compounds. More preferably, it is selected from the group consisting of rapamycin, tacrolimus, and paclitaxel, as well as active analogs or derivatives, such as prodrugs, of these compounds.

According to the invention, the agent is rapamycin. Rapamycin (available under the trade name Rapamune®) is a macrocyclic lactone produced by *Streptomyces hygroscopicus*, found in the soil of Easter Island. Structurally, it resembles tacrolimus and binds to the same target, an intracellular binding protein or immunophilin known as FKBP-12. Accordingly, other molecules which bind this target are also considered. Rapamycin is reported to function by blocking IL2-dependent T-lymphocyte proliferation and the stimulation caused by cross-linkage of CD28, possibly by blocking activation of a serine-threonine kinase that is important for cell cycle progression.

### II. Treatment Method

Restenosis refers to the renarrowing of the vascular lumen following vascular intervention, such as coronary artery balloon angioplasty with or without stent insertion. It is clinically defined as greater than 50% loss of initial luminal diameter gain following the procedure. Restenosis is believed to occur in about 30% to 60% of lesions treated by angioplasty and about 20% of lesions treated with stents within 3 to 6 months following the procedure. (See, *e.g*., Dev).

Stenosis can also occur after a coronary artery bypass operation, wherein heart surgery is done to reroute, or "bypass," blood around clogged arteries and improve the supply of blood and oxygen to the heart. In such cases, the stenosis may occur in the transplanted blood vessel segments, and particularly at the junction of replaced vessels. As noted above, stenosis can also occur at anastomotic junctions created for dialysis.

Disclosed within the context of the present invention are methods for reducing the risk (incidence) or severity (extent) of stenosis, particularly following balloon angioplasty and/or stent implantation, or in response to other vessel trauma, such as following an arterial bypass operation or hemodialysis. As used herein, a "site of vascular injury" or "site of trauma" may be defined as any region of the vessel subjected to excessive pressure, incision, abrasion, or radiation, or other phenomena which would, in the absence of treatment, tend to result in the development of stenosis. Examples include surgical or percutaneous intervention (e.g. PTCA and/or stent implantation) in coronary and/or periperipheral vasculature. Such sites are typically characterized by the presence of damaged vascular endothelium.

More generally, disclosed within the context of the invention are methods to prevent, suppress, or treat hyperproliferative vascular disease. The method includes administering to the affected site, the above-described microbubble- or microparticle-conjugated therapeutic agent(s), in an amount effective to reduce the risk and/or severity of hyperproliferative disease. Administration may take place before, during, and/or after the procedure in question, and multiple treatments may be used. The administration may be via a route such as systemic i.v., systemic intraarterial, intracoronary, *e.g*. via infusion catheter, or intramural, *i.e.* directly to the vessel wall. When the therapeutic agent is rapamycin, preferred doses are typically between about 0.05 - 20 mg/kg, more preferably about 0.1 to 5.0 mg/kg. In another preferred embodiment, about 50-400 mg rapamycin per cm² of affected area is administered.

The therapeutic agents are conjugated to the microparticle carrier, preferably a microbubble composition, alone or in combination. The carrier delivers the agent or agents to the site of vessel damage. Delivery of the compound via the above-described microparticles is effective to achieve high localized concentration of the compound at the vessel injury site, by virtue of adherence of the microparticles to damaged endothelium. In a preferred embodiment, the agent is released at the site without the use of external stimulation. As described below, delivery of rapamycin to a site of vessel injury via microbubbles did not require the use of external ultrasound, nor did it rely on a phase change in the microbubble fluid, as has been described in the prior art. However, if desired, release of the agent may also be modulated by application of a stimulus such as light, temperature variation, pressure, ultrasound or ionizing energy or magnetic field. Application of such a stimulus may also be used to convert a prodrug to the active form of the drug, which is then released.

The use of drug-coated stents has been associated with intrastent (proximal or distal to the stent) stenosis. For example, in a recently conducted multicenter double blind randomized study with rapamycin coated stents (Cordis J&J Bx Velocity), intrastent restenosis was observed in 9% of subjects. This effect is believed to be due to vessel injury that occurs, during stent implantation, in vessel segments adjacent to the stent. Various factors, such as the length of the balloon delivery system (which is always longer than the actual stent), manipulation of the guidewire, and the use of predilation balloon angioplasty, as well as operator error or improper stent length relative to the lesion, can contribute to such injury. In general, no diffusion of drug occurs from a drug-eluting stent (where the drug is, for example, rapamycin, paclitaxel, or tacrolimus) to the adjacent vessel wall, proximal and distal to the implanted stent. Therefore, no antirestonic drug is available in these areas, which are prone to intrastent restenosis phenomena.

A similar effect, known as the edge effect or "candy wrapper" effect, is often observed after vascular brachytherapy (vascular radiation therapy, which often follows balloon angioplasty and/or stent implantation).

Delivery of an antirestenotic compound, as described herein, via the above-described microparticles is thus advantageously used in combination with stent implantation and/or brachytherapy, since the compositions of the invention extend treatment beyond the boundaries of the stent. Microparticle delivery of the drug before treatment, immediately after treatment, or later in time can prevent or reduce the complications described above and greatly improve results obtained from implantation of a drug-eluting (or radiation-emitting) stent. The present method can also be used in applications which are not amenable to stent implantation, e.g. treatment of small or branching vessels, diffuse or bifurcated lesions, or unstable plaque, in addition to treating beyond the boundaries of coated stents.

### III. In vivo Studies

As shown below, rapamycin conjugated to PESDA (perfluorocarbon-exposed sonicated dextrose albumin) microbubbles and administered intravenously showed evidence of penetration into damaged vessels four hours after balloon angioplasty and administration of the composition, and significantly reduced arterial stenosis, in comparison to a control group and a c-myc antisense treated group.

In the study, described in detail in the Materials and Methods section below, seven immature farm pigs were divided into acute and chronic treatment groups. The two acute animals were treated with balloon angioplasty followed by implantation of stents in three separate coronary vessels. One received PESDA microbubbles with rapamycin (2 mg total dose) adsorbed, and the other received PESDA microbubbles with an antisense c*-myc* agent adsorbed. The antisense agent was a phosphorodiamidate-linked morpholino oligomer (see *e.g.* Summerton and Weller, Antisense Nucleic Acid Drug Dev. 7:63-70, 1997) having the sequence 5'-ACGTTGAGGGGCATCGTCGC-3' (SEQ ID NO: 1), which is targeted to the ATG translation site of *c-myc* mRNA (see Iversen and Weller, PCT Pubn. No. WO 00/44897).

### A. Acute Effects

The pigs were sacrificed four hours after treatment, and vessel tissue was examined for expression of p21, p27, β-actin and c-myc. Rapamycin enhances the expression of p21 and p27 and should have no effect on β-actin. The antisense c-myc should inhibit the expression of c-myc, with no effect on β-actin and minimal effect on p21 or p27. Hence, administration of c-myc antisense represents a control for rapamycin treatment, and the rapamycin represents a control for c-myc antisense agent.

Western blot analysis of p21, p27 and β-actin expression was determined by densitometry of bands appearing at the appropriate molecular weight. The band density of p21 relative to β-actin and p27 relative to β-actin are provided in the table below: (LCX= left circumflex artery; LAD = left anterior descending; RCA = right coronary artery)

**Table 1**

| **Vessel** | **p21/β-actin ratio** | | **p27/β-actin ratio** | |
|---|---|---|---|---|
| | **Rap/PESDA** | **PMO/PESDA** | **Rap/PESDA** | **PMO/PESDA** |
| LCX | 0.714 | 0.221 | 1.251 | 0.421 |
| LAD | 1.001 | 0.229 | 3.348 | 1.864 |
| RCA | 0.931 | 0.788 | 0.624 | 0.622 |

These data show that vessels treated with rapamycin carried by microbubbles have elevated expression of both p21 and p27, the anticipated effect of rapamycin. The 2 mg dose in 35 - 40 kg pigs is too small for this effect to be due to systemic accumulation of rapamycin at the injured vessel site. This provides evidence that the microbubbles effectively carry the rapamycin to the site of vessel injury and deposit the rapamycin at the injury site.

### B. Chronic Effects

The remaining 5 pigs were treated with balloon angioplasty and stent implantation, then divided into (1) control (no drug treatment), (2) PESDA/rapamycin treatment and (3) PESDA/antisense c-myc (SEQ ID NO: 1) treatment. Pigs were sacrificed 4 weeks after treatment for analysis of tendency for restenosis. The endpoints for these studies included quantitative angiography and histomorphometry, as described in Materials and Methods below. Histomorphometry data at 28 days post procedure, measured as described in the Examples below, are given in Tables 2 and 3, below.

No evidence of myocardial infarction was seen on gross inspection or after histological evaluation. H&E and VVG-stained sections of all arterial segments were examined. All stents were well developed within the vessel, resulting in thinning of the media adjacent to the stent struts. In the rare vessels with stent protrusion into the adventitia, there was evidence of perivascular hemorrhage. No cases of thrombosis of the treated segment were observed in any of the treatment groups. Complete healing was observed with virtually no toxicity in the treatment groups, and re-endothelialization was complete in all treatment groups.

Neointima from treated arteries was smaller in size than the controls. Control arteries exhibited a substantial neointima, consisting mostly of stellate and spindle-shaped cells, in a loose extracellular matrix. In the antisense treated arteries, the cells of the neointima were morphologically similar to the controls.

Table 2 shows control and rapamycin data for individual vessels. (Note that restenosis reduces the lumen area and increases the intimal and medial area.) Units are in mm and mm².

**Table 2**

| **Vessel - Trtmt** | **Lumen Area** | **Intimal Area** | **Medial Area** |
|---|---|---|---|
| LAD - rapa 661 | 4.62 ± 1.01 | 3.26 ± 2.18 | 1.52 ± 0.31 |
| LAD - rapa 662 | 8.04 ± 1.59 | 2.94 ± 1.26 | 1.85 ± 0.05 |
| LAD - control | 3.55 ± 0.92 | 2.89 ± 0.93 | 1.43 ± 0.18 |
| RCA - rapa 661 | 7.45 ± 0.32 | 1.64 ± 0.55 | 2.08 ± 0.51 |
| RCA - control | 2.54 ± 1.14 | 6.24 ± 1.15 | 1.87 ± 0.42 |
| LCX - rapa 661 | 2.23 ± 1.57 | 3.53 ± 1.40 | 1.02 ± 0.23 |

Both measurements for LAD lumen area are larger in the rapamycin coated microbubble group than in the control groups (4.62 and 8.04 vs. 3.55), and the RCA lumen area is also much larger than in the control (8.04 vs. 2.54). Although, in this study, the rapamycin treatment did not significantly alter medial area or intimal thickening in the LAD, intimal thickening was greatly reduced in the RCA (1.64 vs.6.24).

Table 3 shows averaged histomorphometric data from measurements of the individual vessels. For control, n = 3; for rapamycin, n = 4-6, and for antisense, n = 6. Values for the first ten variables (arterial diameter - lumen area) are in mm or mm². Grading systems described by Kornowski *et al.* and by Suzuki et al. (Circulation 104(10):1188-93, 2001) were used to assess the vessel wall and extent of vascular repair (intimal vascularity; intimal fibrin; intimal SMC content; adventitial fibrosis).

Injury score (IS) and inflammation score were adapted from the scoring system described by Kornowski *et al*., who observed that implanted stents cause neointimal proliferation proportional to injury. The ratio of neointimal area/injury score (IA/IS) provides a normalized value of intimal area related to the extent of vessel injury.

The values of Intimal Thickness and Intimal Area, as well as the normalized values of IA/IS, show that both therapeutic compositions inhibited stenosis relative to the control, with the rapamycin composition significantly superior to the c-myc composition. This is also illustrated in Fig. 1, a regression plot of IA vs. IS for the three treatment groups.

**Table 3**

| **Variable** | **Control** | **Rapamycin** | **c-myc Antisense** |
|---|---|---|---|
| Arterial Area | 9.70 ± 1.58 | 10.04 ± 2.59 | 10.94 ± 2.09 |
| **Intimal Area (IA)** | **4.77 ± 1.71** | **1.84 ± 0.44** | **2.83 ± 1.99** |
| Media Area | 1.60 ± 0.24 | 1.62 ± 0.46 | 1.83 ± 0.45 |
| Int/Med Ratio | 3.02 ± 0.80 | 2.11 ± 1.25 | 1.81 ± 1.59 |
| Lumen Area | 3.34 ± 0.72 | 6.55 ± 1.69 | 6.07 ± 3.20 |
| Area % Occl. | 57.53 ± 13.19 | 26.00 ± 19.00 | 33.26 ± 24.63 |
| Lum/Art Ratio | 0.35 ± 0.11 | 0.65 ± 0.16 | 0.55 ± 0.20 |
| **Injury Score (IS)** | 1.92 ± 0.63 | 1.75 ± 0.46 | 1.13 ± 0.96 |
| **IA/IS** | **2.48** | **1.05** | **2.50** |
| Inflam Score | 0.67 ± 0.52 | 0.44 ± 0.13 | 0.17 ± 0.30 |
| Intimal Vascularity | 0.42 ± 0.52 | 0.38 ± 0.48 | 0.17 ± 0.30 |
| Intimal Fibrin | 0.17 ± 0.14 | 0.19 ± 0.24 | 0.21 ± 0.25 |
| Intimal SMC Content | 3.00 ± 0.00 | 3.00 ± 0.00 | 3.00 ± 0.00 |
| Adventitial Fibrosis | 1.17 ± 0.76 | 0.88 ± 0.25 | 0.71 ± 0.62 |

| | | | |
|---|---|---|---|
| IEM = internal elastic lamina; SMC = smooth muscle cell | | | |

### Materials and Methods

### Rapamycin/PESDA

PESDA microbubbles were prepared as described in, for example, U.S. Patent No. 6,245,747 and PCT Pubn. No. WO 2000/02588. In a typical procedure, 5% human serum albumin and 5% dextrose, obtained from commercial sources, are drawn into a 35 mL syringe in a 1:3 ratio, hand agitated with 6-10 mL of decafluorobutane, and sonicated at 20 kilohertz for 75-85 seconds. As described in U.S. 6,245,747, the mean size of four consecutive samples of PESDA microbubbles produced in this manner, as measured with hemocytometry, was 4.6±0.4 microns, and mean concentration, as measured by a Coulter counter, was 1.4x10⁹ bubbles/mL.

A solution of rapamycin in a pharmaceutically acceptable solvent, such as alcohol, DMSO, or castor oil, was incubated with agitation with the PESDA microbubble suspension at room temperature. The mixture was allowed to settle, with the rapamycin-conjugated microbubbles rising to the top. If necessary, the rapamycin solution is sterilized and/or filtered through a micropore filter prior to incubation.

### Animals and Experimental Protocol

Animals received humane care in compliance with the "Principles of Laboratory Animal Care" formulated by the National Society for Medical Research and the "Guide for the Care and Use of Laboratory Animals" prepared by the National Academy of Sciences and published by the National Institutes of Health (NIH publication #85-23, revised 1985).

Seven female or male juvenile pigs (25 to 30 kg) were sedated with a combination of ketamine (20 mg/kg) and xylazine (2 mg/kg) by intramuscular injection. The animals were given pentobarbital (10-30 mg/kg IV) and were subsequently intubated and ventilated with oxygen (2 L/min) and isoflurane 1% (1.5 L/min) using a respirator. Adequate anesthesia was confirmed by the absence of a limb withdrawal reflex. Limb-lead electrocardiography and blood pressure (Honeywell E for M) were monitored throughout the procedure.

After placement of an 8F-introducer sheath in the right carotid artery by surgical cutdown, each animal received heparin (150 units/kg). Under fluoroscopic guidance, an 8F guiding catheter was positioned in the left or right coronary ostium. Coronary angiography was performed after intracoronary nitroglycerin (200 µg) administration and recorded on cine film (Phillips Cardiodiagnost; Shelton, CT).

### Stent implantation

Coronary stenting was performed at the site of delivery using V-Flex stents 15 mm in length (Cook Inc., Bloomington, IN), hand crimped on the balloon and deployed at high pressure (10-14 Atm X 30 sec). The stents were mounted on a balloon 3.5-4.0 mm in diameter and 20 mm in length. The stent artery ratio was kept between 1:1.1 - 1:1.2. Immediately postprocedure, angiograms were performed to assess vessel patency; the carotid sheath was removed, the carotid artery ligated, the skin closed and the animal allowed to recover. All animals were pretreated with aspirin 325 mg and ticlopidine 250 mg BID, 24 hours prior to the procedure until sacrifice.

### Efficacy of rapamycin delivery into tissues

To evaluate the impact of rapamycin delivery upon p21 and p27 expression following stent implantation, two juvenile pigs weighing 30-35kg underwent oversized multiple stent implantation (3 per animal) in the coronary artery. This was followed by i.v. injection of rapamycin/PESDA complex (2 mg rapamycin). Four hours after the procedure, the pigs were sacrificed, and injured tissues were analyzed by western blot for p21 and p27 expression.

### Chronic studies

The remaining 5 pigs were treated with balloon angioplasty and stent implantation, as described above, and divided into (1) control (no drug treatment), (2) rapamycin/PESDA treatment (2 mg rapamycin) and (3) antisense c-myc/PESDA treatment. At four weeks the animals were sacrificed. The arteries were perfusion-fixed and the injured segments, located with the guidance of the coronary angiograms, were dissected free from the heart. The segment was fixed in 10% formalin solution and embedded in paraffin or a cold polymerizing resin (Technovit 7100; Heraus Kulzer GmbH, Wehrheim, Germany). Cross-sections (5 µm) were stained with hematoxylin and eosin (H&E) and Verhoeff van-Giesson elastin (WG) stain.

### Histological and Morphometric Analysis

Histomorphometric analysis was performed on each segment with evidence of medial fracture. The histomorphometric parameters were measured on 5-8 sections per vessel, averaged and expressed as mean value ± SD. Vessel sections were measured by an experienced investigator who was unaware of the treatment group assignment.

The histopathological features were measured using a computerized PC-compatible image analysis program (Optimas 6; Optimas, Inc., Bothell, WA). VVG-stained sections were magnified at 7.5 X, digitized, and measured in a frame-grabber board (DAGE-MTI, Michigan City, IN). Area measurements were obtained by tracing the lumen perimeter (luminal area, LA, mm²), medial perimeter (medial area, MA, mm²), neointima perimeter (intimal area, IA, mm², defined by the borders of the internal elastic lamina, lumen, media, and external elastic lamina), and external elastic lamina (vessel area, VA, mm²).

Injury score and inflammation score were adapted from the scoring system described by Kornowski et al., J. Am. Coll. Cardiol. 31:224-30 (1998), and the grading scheme of Kornowski *et al.* and Suzuki et al. (Circulation 104(10):1188-93, 2001) was used to assess the vessel wall and extent of vascular repair.

Endothialization was scored on the basis of percent of the intimal surface covered by endothial cells: (1) 0-25%; (2) 25-75%, and (3) >75%.

Intimal fibrin content was graded based on the following criteria: (1) focal residual fibrin involving any portion of the artery; moderate fibrin deposition adjacent the stent strut involving <25% of the circumference of the vessel; (2) moderate fibrin deposition involving >25% of the circumference of the vessel; (3) heavy fibrin deposition involving <25% of the circumference of the vessel.

Intimal SMC content was graded based on the following criteria: (1) sparse SMC density involving any portion of the artery; moderate SMC infiltration less than the full thickness of the neointima involving <25% of the circumference of the vessel; (2) moderate SMC infiltration less than the full thickness of the neointima involving >25% of the circumference of the vessel or dense SMC content the full thickness of the neointima involving <25% of the circumference of the vessel; (3) dense SMC content the full thickness of the neointima involving >25% of the circumference of the vessel.

After artery removal, hearts were sectioned transaxially at 1cm intervals and examined for evidence of myocardial damage.

### Statistical Evaluation

Data (mean ± standard deviation) were analyzed for overall differences between treatment groups using one-way ANOVA with the Bonferroni correction. Comparison of the mean values with a p value of less than 0.05 was considered statistically different. All statistics were performed using SPSS 10.0 for Windows (SPSS, Inc. Chicago, Illinois). The intimal area and injury score were correlated using linear regression analysis.

## Claims

1. Use of a composition comprising an antirestenotic compound non-covalently conjugated to a microparticle carrier for the preparation of a pharmaceutical composition for inhibiting stenosis formation at a site of trauma in a blood vessel, wherein the antirestenotic compound is rapamycin and the microparticle carrier comprises a suspension of insoluble gas-containing microbubbles in a pharmaceutically acceptable liquid vehicle.

2. Use of claim 1, wherein the pharmaceutical composition is to be administered prior to, during, and/or following a procedure selected from balloon angioplasty, stent implantation, and surgical incision or grafting of the vessel.

3. Use of claim 2, wherein the procedure comprises balloon angioplasty and/or stent implantation.

4. Use of claim 3, wherein the procedure comprises stent implantation, and said administration is effective to inhibit intrastent restenosis.

5. Use of claim 1, wherein the pharmaceutical composition is to be administered without application of external stimulation to said composition during or following administration.

6. Use of claim 1, wherein the composition further comprises, non-covalently conjugated to the microparticle carrier, an antiinflammatory compound, a compound effective to inhibit collagen accumulation or calcification of the vascular wall or a combination thereof.

7. Use of claim 1, wherein the microparticle carrier comprises an aqueous suspension of insoluble gas-containing microbubbles.

8. Use of claim 7, wherein the gas is SF₆ or a perfluorocarbon gas.

9. Use of claim 8, wherein the gas is selected from perfluoromethane, perfluoroethane, perfluoropropane, perfluorobutane, and perfluoropentane.

10. Use of claim 7, wherein the aqueous suspension contains at least one filmogenic compound selected from a protein, surfactant, lipid, polysaccharide, and combinations thereof.

11. Use of claim 10, wherein the aqueous suspension comprises human serum albumin and dextrose.

12. Use of a composition comprising an antirestenotic compound non-covalently conjugated to a microparticle carrier, wherein the microparticle carrier comprises a suspension of insoluble gas-containing microbubbles in a pharmaceutically acceptable liquid vehicle for the preparation of a pharmaceutical composition for inhibiting intrastent stenosis formation, at regions proximal and distal to an implanted stent in a blood vessel, wherein intrastent stenosis formation is inhibited and wherein the antirestenotic compound is rapamycin.

13. Use of claim 12, wherein the pharmaceutical composition is to be administered prior to, during, and/or following implantation of the stent.

14. Use of claim 12, wherein the pharmaceutical composition is to be administered without application of external stimulation to said composition during or following administration.

15. Use of claim 12, wherein the microparticle carrier comprises a suspension of perfluorocarbon gas-containing microbubbles in an aqueous vehicle.

## Patentansprüche

1. Verwendung einer Zusammensetzung, umfassend eine Verbindung gegen Restenose, die nichtkovalent an einen Mikropartikelträger gebunden ist, zur Herstellung einer pharmazeutischen Zusammensetzung zur Verhinderung der Entwicklung von Stenose an einer Verletzungsstelle in einem Blutgefäß, wobei die Verbindung gegen Restenose Rapamycin ist und der Mikropartikelträger eine Suspension von Mikrobläschen, die ein unlösliches Gas enthalten, in einem pharmazeutisch verträglichen flüssigen Vehikel umfasst.

2. Verwendung nach Anspruch 1, wobei die pharmazeutische Zusammensetzung zu verabreichen ist vor, bei und/oder nach einem Verfahren, ausgewählt aus Ballon-Angioplastie, Stent-Implantation und operativem Schnitt oder Transplantation des Gefäßes.

3. Verwendung nach Anspruch 2, wobei das Verfahren Ballon-Angioplastie und/oder Stent-Implantation umfasst.

4. Verwendung nach Anspruch 3, wobei das Verfahren Stent-Implantation umfasst, und die Verabreichung wirksam ist, um Restenose innerhalb des Stents zu verhindern.

5. Verwendung nach Anspruch 1, wobei die pharmazeutische Zusammensetzung zu verabreichen ist ohne Anwendung von äußerer Stimulation auf die Zusammensetzung während oder nach der Verabreichung.

6. Verwendung nach Anspruch 1, wobei die Zusammensetzung weiterhin umfasst, nichtkovalent an den Mikropartikelträger gebunden, eine entzündungshemmende Verbindung, eine Verbindung, die wirksam ist, um Kollagenansammlung oder Verkalkung der Gefäßwand zu verhindern, oder eine Kombination davon.

7. Verwendung nach Anspruch 1, wobei der Mikropartikelträger eine wässrige Suspension von Mikrobläschen, die ein unlösliches Gas enthalten, umfasst.

8. Verwendung nach Anspruch 7, wobei das Gas SF₆ oder ein Perfluorkohlenwasserstoffgas ist.

9. Verwendung nach Anspruch 8, wobei das Gas ausgewählt ist aus Perfluormethan, Perfluorethan, Perfluorpropan, Perfluorbutan und Perfluorpentan.

10. Verwendung nach Anspruch 7, wobei die wässrige Suspension mindestens eine filmbildende Verbindung, ausgewählt aus einem Protein, einem grenzflächenaktiven Stoff, einem Lipid, einem Polysaccharid, und Kombinationen davon, enthält.

11. Verwendung nach Anspruch 10, wobei die wässrige Suspension humanes Serumalbumin und Dextrose umfasst.

12. Verwendung einer Zusammensetzung, umfassend eine Verbindung gegen Restenose, die nichtkovalent an einen Mikropartikelträger gebunden ist, wobei der Mikropartikelträger eine Suspension von Mikrobläschen, die ein unlösliches Gas enthalten, in einem pharmazeutisch verträglichen flüssigen Vehikel umfasst, zur Herstellung einer pharmazeutischen Zusammensetzung zur Verhinderung der Entwicklung von Stenose innerhalb des Stents, in Bereichen proximal und distal zu einem implantierten Stent in einem Blutgefäß, wobei die Entwicklung von Stenose innerhalb des Stents verhindert wird und wobei die Verbindung gegen Restenose Rapamycin ist.

13. Verwendung nach Anspruch 12, wobei die pharmazeutische Zusammensetzung zu verabreichen ist vor, während und/oder nach der Implantation des Stents.

14. Verwendung nach Anspruch 12, wobei die pharmazeutische Zusammensetzung zu verabreichen ist ohne Anwendung von äußerer Stimulation auf die Zusammensetzung während oder nach der Verabreichung.

15. Verwendung nach Anspruch 12, wobei der Mikropartikelträger eine Suspension von Mikrobläschen, die ein Perfluorkohlenwasserstoffgas enthalten, in einem wässrigen Vehikel umfasst.

## Revendications

1. Utilisation d'une composition comprenant un composé anti-resténose non conjugué de manière covalente à un support de micro-particules pour la préparation d'une composition pharmaceutique pour inhiber la formation de sténose sur un site de traumatisme dans un vaisseau sanguin, dans lequel le composé anti-resténose est la rapamycine et le support de micro-particules comprend une suspension de microbulles contenant un gaz insoluble dans un véhicule liquide pharmaceutiquement acceptable.

2. Utilisation selon la revendication 1, dans laquelle la composition pharmaceutique doit être administrée avant, pendant, et/ou à la suite d'une procédure choisie parmi une angioplastie par ballonnet, une implantation de stent, et une incision chirurgicale ou une greffe du vaisseau.

3. Utilisation selon la revendication 2, dans laquelle la procédure comprend une angioplastie par ballonnet et/ou une implantation de stent.

4. Utilisation selon la revendication 3, dans laquelle la procédure comprend une implantation de stent, et ladite administration est efficace pour inhiber la resténose intrastent.

5. Utilisation selon la revendication 1, dans laquelle la composition pharmaceutique doit être administrée sans application de stimulation externe à ladite composition pendant ou à la suite de l'administration.

6. Utilisation selon la revendication 1, dans laquelle la composition comprend en outre, non conjugué de manière covalente au support de micro-particules, un composé anti-inflammatoire, un composé efficace pour inhiber l'accumulation de collagène ou la calcification de la paroi vasculaire ou une combinaison de celles-ci.

7. Utilisation selon la revendication 1, dans laquelle le support de micro-particules comprend une suspension aqueuse de microbulles contenant un gaz insoluble.

8. Utilisation selon la revendication 7, dans laquelle le gaz est du SF₆ ou un gaz de perfluorocarbone.

9. Utilisation selon la revendication 8, dans laquelle le gaz est choisi parmi le perfluorométhane, le perfluoroéthane, le perfluoropropane, le perfluorobutane et le perfluoropentane.

10. Utilisation selon la revendication 7, dans laquelle la suspension aqueuse contient au moins un composé filmogène choisi parmi une protéine, un agent tensio-actif, un lipide, un polysaccharide, et des combinaisons de ceux-ci.

11. Utilisation selon la revendication 10, dans laquelle la suspension aqueuse comprend de l'albumine sérique humaine et du dextrose.

12. Utilisation d'une composition comprenant un composé anti-resténose non conjugué de manière covalente à un support de microparticules, dans laquelle le support de micro-particules comprend une suspension de microbulles contenant un gaz insoluble dans un véhicule liquide pharmaceutiquement acceptable pour la préparation d'une composition pharmaceutique pour inhiber la formation de sténose intrastent, au niveau de régions proximale et distale à un stent implanté dans un vaisseau sanguin, dans laquelle la formation de sténose intrastent est inhibée et le composé anti-resténose est la rapamycine.

13. Utilisation selon la revendication 12, dans laquelle la composition pharmaceutique est administrée avant, pendant et/ou à la suite de l'implantation du stent.

14. Utilisation selon la revendication 12, dans laquelle la composition pharmaceutique doit être administrée sans application de stimulation externe à ladite composition pendant ou à la suite de l'administration.

15. Utilisation selon la revendication 12, dans laquelle le support de micro-particules comprend une suspension de microbulles contenant un gaz de perfluorocarbone dans un véhicule aqueux.


### SEQUENCE LISTING

<110> AVI BioPharma, Inc.
   Iversen, Patrick L.
   Kipshidze, Nicholas
<120> DELIVERY OF MICROPARTICLE-CONJUGATED DRUGS FOR INHIBITION OF STENOSIS
<130> 504508318WO0
<140> PCT/US03/13892
   <141> 2003-05-02
<150> US 10/138,589
   <151> 2002-05-03
<150> US 10/190,419
   <151> 2002-07-02
<160> 1
<170> PatentIn Ver. 2.1
<210> 1
   <211> 20
   <212> DNA
   <213> Artificial Sequence
<220>
   <223> Description of Artificial Sequence: phosphorodiamidate-linked morpholino oligomer
<400> 1
   acgttgaggg gcatcgtcgc 20